# EUROPEAN PATENT SPECIFICATION

(11) **EP 3 970 183 B1**
(45) Date of publication and mention of the grant of the patent: **09.07.2025**
(21) Application number: 21770110.1
(22) Date of filing: 20.04.2021
(51) Int. Cl.: H01J 49/34, G01N 29/02, G01N 15/10, G01N 33/543, G01G 3/16, G01N 33/68, G01N 29/036

(54) **DEVICE FOR OBTAINING THE MASS OF SINGLE NANOPARTICLES, VIRUSES AND PROTEINS IN SUSPENSION OR IN SOLUTION WITH HIGH-COLLECTION EFFICIENCY**
VORRICHTUNG ZUR GEWINNUNG DER MASSE EINZELNER NANOPARTIKEL, VIREN UND PROTEINE IN SUSPENSION ODER IN LÖSUNG MIT HOHEM SAMMELEFFIZIENZ
DISPOSITIF PERMETTANT D'OBTENIR LA MASSE DE NANOPARTICULES, DE VIRUS ET DE PROTÉINES UNIQUES EN SUSPENSION OU DANS UNE SOLUTION AVEC UNE EFFICACITÉ DE COLLECTE ÉLEVÉE

(30) Priority: 24.07.2020 US 202063055917 P; 10.04.2021 US 202117227306
(43) Date of publication of application: 23.03.2022
(73) Proprietor: Ihsan Dogramaci Bilkent Üniversitesi, 06800 Ankara (TR)
(72) Inventor: HANAY, Mehmet Selim, 06800 Cankaya/Ankara (TR); ERDOGAN, Ramazan Tufan, 06800 Cankaya/Ankara (TR); ALKHALED, Mohammed, 06800 Cankaya/Ankara (TR); PISHEH, Hadi Sedaghat, 06800 Cankaya/Ankara (TR)
(74) Representative: Sevinç, Cenk
(86) International application number: PCT/TR2021/050363
(87) International publication number: WO 2022/019855

(56) References cited:
- WO-A1-2011/060369
- US-A1- 2011 186 167
- US-A1- 2013 238 252
- US-A1- 2018 005 809
- US-B2- 9 121 823
- A. K. NAIK ET AL: "Towards single-molecule nanomechanical mass spectrometry", NATURE NANOTECHNOLOGY, vol. 4, no. 7, 21 June 2009 (2009-06-21), pages 445 - 450, XP055024639, ISSN: 1748-3387, DOI: 10.1038/nnano.2009.152
- SERGIO DOMINGUEZ-MEDINA ET AL: "Charge-independent mass spectrometry of single virus capsids above 100MDa with nanomechanical resonators", ARXIV.ORG, CORNELL UNIVERSITY LIBRARY, 201 OLIN LIBRARY CORNELL UNIVERSITY ITHACA, NY 14853, 6 April 2018 (2018-04-06), XP081226197

## Description

### Technical Field of the Invention

The present invention relates to a device for determining the mass of a nanoparticle, virus or protein in a suspension or solution in a fluid. This device can be applied in particular to mass spectrometry for ionized species with high collection efficiency (i.e. low limit of detection).

### Background of the Invention (Prior Art)

Physical methods to measure the mass of species in the range of 100kDa to 1000 MDa with single molecule resolution have been reported scarcely in the state of art. Commercial Mass Spectrometry has a cut-off for species heavier than several MegaDalton molecular mass, due to detector limitations. In the last decade, a new approach based on miniature mechanical resonators has been developed to measure the mass of species landing on the resonator. Since this miniature mechanical resonator has taken the form of a Nano-Electromechanical System (NEMS) for the significant demonstrations so far, this approach has been named as NEMS-Mass Spectrometry (NEMS-MS). NEMS-MS has been shown to work successfully on proteins such as Bovine BSA and antibodies such as human IgM.

In NEMS-MS, when the species land on the structure, it increases the effective mass of the resonator, and as a result, the resonance frequency of the resonator shifts down abruptly. By using the resonance frequency shifts, the mass of the species can be determined in one method, by statistically collecting many identical particles. In the study of A. K. Naik et al. [1], the first demonstration of mass spectrometry based on single biological molecule detection with a nanoelectromechanical system has been reported. In that nanoelectromechanical-mass spectrometry system, nanoparticles and protein species are introduced by electrospray injection from the fluid phase in ambient conditions into vacuum and are subsequently delivered to the nanoelectromechanical system detector by hexapole ion optics.

In another NEMS-MS method, the mass of the particle can be determined by the simultaneous tracking of several mechanical modes, such as the first two flexural mode of a doubly-clamped beam. M.S. Hanay, et al. [2], has demonstrated the first realization of single-molecule NEMS-based mass spectrometry in real time. Herein, as each molecule in the sample adsorbs on the resonator, its mass and position of adsorption are determined by continuously tracking two driven vibrational modes of the device. They have demonstrated the potential of multimode NEMS-based mass spectrometry by analyzing IgM antibody complexes in real time.

In the references cited above, the NEMS detector was placed in an ultra-high vacuum chamber to increase its sensitivity. On the other hand, the species to be analyzed started as solvents in a water-based solution and were then subsequently converted into gas phase ions using Electrospray Ionization (ESI) technique, which is a commonly used technique especially in association with conventional mass spectrometry. Due to the vast difference in the pressures between the Electrospray Ionization condition and NEMS chamber, the aforementioned references employed a differential vacuum system and ion guides to transport the ionic species onto the NEMS chips. Due to the inherent losses in the ion guides, apertures between different differential pressure chambers, and the small cross section of the NEMS detector, the collection efficiency of the species has been very small.

Some methods were developed to increase the detection efficiency of the NEMS devices. In one case, the NEMS detector was placed at a chamber closer, both in terms of pressure and distance, to the Electrospray Ionization source. In the study of O. Malvar, et al. [3], it has been demonstrated that heavier analytes can be identified by their mass and stiffness by using nanomechanical resonators. In this study, they have performed nanomechanical spectrometry of 100 nm-sized gold nanoparticles (GNPs) and Escherichia coli DH5α cells using microcantilever resonators. They have developed theoretical methods that enable the determination of the stiffness, mass and position of the analytes arriving the microcantilever from the resonance frequency jumps. Ignoring the effect of the stiffness leads to an underestimation of the mass of 10% for the used microcantilevers. In this case, less than one particle per 10⁹ particles in solution was detected. To increase the collection efficiency of NEMS detectors, another approach was developed that is based on aerodynamic lensing effect in the patent no. US 9,506,852 B2 which discloses a device for determining the mass of at least one particle in suspension or in solution in a fluid.

In the study of S. Dominguez-Medina, et al. [4], it has been reported that a system architecture combining nebulization of the analytes from solution, their efficient transfer and focusing without relying on electromagnetic fields, and the mass measurements of individual particles using nanomechanical resonator arrays. In this system, only one virus particle detected per 2.6 x 10⁸ particles in the solution. However, the concentration of virus particles in realistic samples are almost always smaller than 2.6 × 10⁸ per mL of fluid, with 1 mL being the typical volume sampled from patients. The typical ranges for virus concentration from human samples are between 10³ virus per mL to 10⁶ virus per mL, due to the study of J.D. Spitzberg, et al. [5]. Hence, the collection efficiency of the techniques involving NEMS-MS are not sufficient to be used for screening for viral infection in humans. The aerodynamic lensing approach still creates a focus size on the order of millimeter. To obtain a flux significant enough to work with clinical human samples within a reasonable time, the particle flux needs to be focused much more tightly: in the ideal case, the spot size should match the capture cross section of the NEMS sensor.

Another shortcoming of the previous NEMS-MS approaches, as the technology is applied to virus detection for population screening, is that they require the NEMS detector to operate under high or ultra-high vacuum conditions, which necessitates material and equipment with higher costs. Finally, in current NEMS-MS systems, the NEMS chip is needed to be placed on a micro/nano-positioning system to move the active NEMS sensor to the region where maximum ion flux is delivered. However, this approach both increases the cost of the equipment as well as increases the total analysis time, since for each NEMS device or array introduced, a search procedure should be implemented to find the location of maximum intensity. Clearly, a focusing device that is close to the NEMS detector and fabricated in such a way that the focused particle beam is already aligned with the NEMS detector would form a much more efficient particle collection approach.

### Summary of the Invention

The present invention relates to a device for determining the mass of single nanoparticles, viruses and proteins in suspension or in solution with high-collection efficiency. The device comprises a first device for creating charged particles of interest in gas phase; a second device for determining the mass of the particle by a frequency measurement comprising at least one gravimetric detector; a third device that is fabricated on the same chip with, and surrounding the second device to focus and guide the majority of the incoming charged particles including at least the particle by means of holding charge on itself to act as an electrostatic lens.

The aim of the present invention is to increase the collection efficiency of NEMS-MS approach such that NEMS-MS can be used in realistic situations, for instance, but not limited to, the detection and mass spectrometric identification of virus particles in a sample obtained from a human.

Another aim of the present invention is to determine the mass of each particle that lands on the subject-matter of device. The collection efficiency is defined as the number of particles arriving at the NEMS detector for analysis over the number of particles originally resided in the sample and subsequently utilized during the process. The collection efficiency is increased for charged analytes by fabricating or placing a sufficiently insulating layer of material near the NEMS detector. Since the focusing layer is already co-fabricated with the NEMS detector, there is no need for a separate alignment procedure for the NEMS and the focusing device. The device subject to the invention, thus makes it possible to determine the mass of each particle that lands on the device and increases the particle flux received by the second device, owing to the electrostatic focusing effect of the third device.

Another aim of the present invention is to decrease total cost of the system to determine the mass of single nanoparticles, viruses and proteins. According to a particular embodiment, the device is situated in ambient pressure. The advantage of this embodiment is that total cost of the system decreases as there is no vacuum-related equipment.

### Brief Description of the Drawings

The present invention will be better understood on reading the description of embodiment examples given hereafter, purely as an indication and in no way limiting, and by referring to the appended drawings in which:
**Figure 1** is a schematic view of a particular embodiment of the device, the subject matter of the invention.
**Figure 2** is a schematic view of a particular embodiment of the device, the subject matter of the invention, supplemented with various auxiliary mechanisms to increase the performance of the device.
**Figure 3** is a drawing as an example of the chip surface holding device 2 and device 3.
**Figure 4** is a schematic view of a particular embodiment of the device for determining the mass of single nanoparticles, viruses and viruses in a suspension or solution in a fluid with high collection efficiency.
**Figure 5** is a Scanning Electron Micrograph of a NEMS device fabricated within an empty window on a photoresist layer, wherein the insulating photoresist layer in this case acts as a self-biased electrostatic lens.
**Figure 6** is a top-down view of a Scanning Electron Micrograph of another NEMS device fabricated within an empty window on a photoresist layer, wherein the insulating photoresist layer in this case acts as a self-biased electrostatic lens.
**Figure 7** shows an optical micrograph of a NEMS sensor device fabricated within an electrostatic lensing windows formed by photoresist (In this figure, some particles have already been deposited by electrospray ionization, as a result a halo-like accumulation near the window and blurred features on the NEMS sensor and other places inside the focusing window.).
**Figure 8** shows a Scanning Electron micrograph of a NEMS sensor device fabricated within an electrostatic lensing windows formed by photoresist, wherein individual nanoparticles are discernible in this micrograph and the nanoparticles are gold nanoparticles with a nominal diameter of 20 nm.
**Figure 9** shows a Scanning Electron micrograph of a NEMS sensor device fabricated within an electrostatic lensing windows formed by photoresist, wherein individual nanoparticles are discernible in this micrograph and the nanoparticles are gold nanoparticles with a nominal diameter of 20 nm.
**Figure 10** shows a closed-up view of a Scanning Electron micrograph of a NEMS sensor device fabricated within an electrostatic lensing windows formed by photoresist, wherein individual nanoparticles are clearly visible in this micrograph; and moreover, the lensing effect is seen as the number density of nanoparticles decrease away from the center of the focusing window in the horizontal direction. The nanoparticles are gold nanoparticles with a nominal diameter of 20 nm.
**Figure 11** shows a closed-up view of a Scanning Electron micrograph of the transduction electrode part of the NEMS sensor device fabricated within an electrostatic lensing windows formed by photoresist, wherein individual nanoparticles are clearly visible in this micrograph; and moreover, the lensing effect is seen as the number density of nanoparticles decrease away from the center of the focusing window in the vertical direction, and the nanoparticles are gold nanoparticles with a nominal diameter of 20 nm.
**Figure 12** is the output of the NEMS sensor in the form of frequency change as a function of time, wherein each sudden downward shift indicates the arrival and detection of a single gold nanoparticle.
**Figure 13** shows an optical micrograph of a NEMS sensor device fabricated within an electrostatic lensing windows formed by photoresist, wherein individual nanoparticles and nanoparticle aggregates are discernible in this micrograph and the nanoparticles are polystyrene nanoparticles, incorporating fluorescent dye molecules, and also the nominal diameter for nanoparticles is 100 nm.

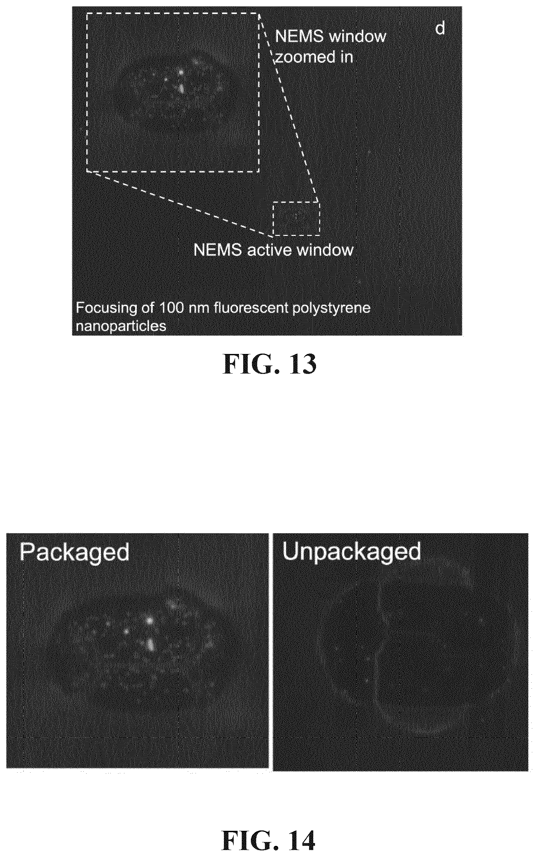

**Figure 14** shows two optical micrographs of two similar NEMS sensor devices fabricated within an electrostatic lensing windows formed by photoresist. 100 nm diameter, fluorescent polystyrene nanoparticles are delivered to both chips under identical conditions. The left sensor shows the case where the bonding pads are covered by an insulating material through packaging techniques, whereas the right sensor shows the case of the unpackaged sensor with bonding pads left open. The difference in collection efficiencies show the importance of packaging as any region left open on the surface due to incomplete packaging will decrease lensing efficiency.

### Description of References in Drawings

The references are presented below:
**1.** First device
**2.** Charges
**3.** Insulating layer
**4.** Gravimetric detector
**5.** Substrate
**6.** Platform
**7.** Layer
**8.** Charge-generation source
**9.** Voltage source
**10.** Sheath gas
**11.** Drying gas
**12.** Lens / conductive device
**13.** Opening
**14.** Collection of electrodes
**15.** Tube

### Detailed Description of the Invention

The present invention device comprises a first device (1) for creating charged particles of interest in gas phase, a second device for determining the mass of the particle by a frequency measurement comprising at least one gravimetric detector (4) (mass sensor) produced on a chip and a third device that is fabricated on the same chip with second device, and surrounding the second device to focus and guide the majority of the incoming charged particles including at least the particle by means of holding charge on itself to act as an electrostatic lens. The charge on the third device can be induced either by the original electrospray of the same polarity as the particle itself or by a separate mechanism such as, including but not limited to, by using a separate tip to generate charging through a proper mechanism such as electrospray or corona discharging.

Preferably, the subject-matter of the invention, the device further comprises a separate electrostatic lens (12) in the free space between the first device (1) and the third device for coarse focusing of the charged particles and shielding the second device from adverse effects of the first device (1), such as electrical arcing, a gas flow nearly or perfectly parallel with the electrospray direction for further focusing the species, auxiliary gas flows to facilitate the evaporation of charged droplets, a voltage applied to substrate (5) of the second device either directly or through a printed circuit board to bias the substrate (5) of the second device with respect to the first device (1).

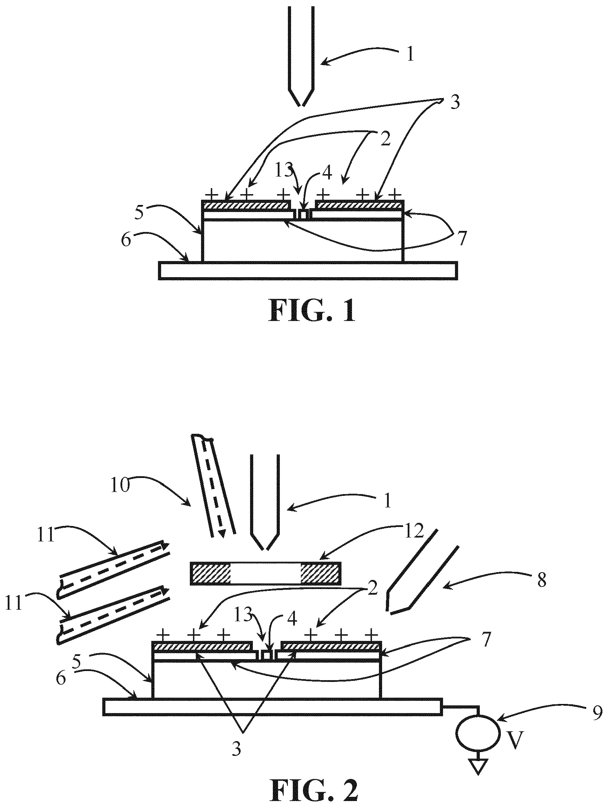

The first device (1) can be any device that ionizes the molecules in the sample to be analyzed without degradation. In other words, the first device (1) is an ionization source. In an embodiment of the invention (Fig.1), first device (1) is an electrospray ionization source and comprises a capillary tube with a pointed tip, sample solution, high voltage source, fittings required to pass the sample solution through the capillary tube and pumps.

According to a particular embodiment, the device is situated in ambient pressure. The advantage of this embodiment is that total cost of the system decreases as there will be no vacuum-related equipment in the subject-matter of device.

According to a particular embodiment, the first device (1) is situated at ambient pressure, while the second and third devices can be situated in a high-vacuum or ultra-high vacuum chamber. The ion transportation in between the chambers can be accomplished by aerodynamic and ion optics means.

According to a particular embodiment, the first device (1) is an electrospray ionization source with a tip radius small enough to sustain and electrospray into a low-vacuum chamber in which the first device is housed. In this embodiment, the second and third devices are situated in a high-vacuum or ultra-high vacuum chamber. The ion transportation in between can be accomplished by aerodynamic and ion optics means.

According to a particular embodiment, the first device (1) is a source that can operate at high vacuum and ultrahigh vacuum conditions such as Electrohydrodynamic Ionization, MALDI (Matrix Assisted Laser-Desorption Ionization), or LIAD (Laser Induced Acoustic Desorption). In this embodiment, the second and third devices are situated in the same vacuum chamber with the first device.

The second device has at least one gravimetric detector (4) which may be selected from nano-electromechanical systems, micro-electromechanical systems, quartz crystal microbalances, surface acoustic resonators, bulk acoustic resonators, impact detectors, and resonant microwave detectors. It is understood that the output of the second device is a physical signal proportional to the mass of a particle adsorbed on its surface as in the technique of NEMS-MS.

The first device (1) may be selected from Electrospray Ionization, Electrohydrodynamic Ionization, MALDI (Matrix Assisted Laser-Desorption Ionization), LIAD (Laser Induced Acoustic Desorption) sources, ultrasonic nebulizers, microwave induced nebulization devices, microcapillary array nebulizers, surface acoustic wave nebulizers. The first device (1) may be supplemented by an auxiliary technique to introduce additional charges on the droplets such as field emission, or corona discharge.

The third device is an insulating layer (3), that surrounds the rest of the chip, leaving the mechanical sensor mentioned in the second device empty. The important feature of this insulating layer (3) is to accumulate electrical charge on it, allowing the sample ions sent by the first device (1) to focus on the mechanical sensor. This insulating layer (3) can be almost on the same level (on) or above (above) the level of the mechanical sensor.

The third device comprises an insulating layer (3), to hold the incoming charges and an opening (13) on the insulating layer (3) aligned with the second device, through which incoming charged ions are focused. The third device may be formed on a layer (7) that is just on the top of the layer (7) that contains the second device and may be out of any sufficiently insulating material, for instance polymers, photoresists, dielectrics such as Silicon Dioxide or Silicon Nitride. The third device has a thickness of 20 nm to 1 mm so that the charged accrued on the third device does not get neutralized quickly: this way electrostatic lensing effect can form.

FIG. 1 is a schematic view of a particular embodiment of the device for determining the mass of single nanoparticles, viruses and proteins in a suspension or solution in a fluid with high collection efficiency. Said device comprises:
- a first device (1) which is electrospray ionization source for electro-spraying the fluid, to generate charged droplets containing the analyte particles and to obtain a charged flux comprising at least the particle,
- a second device for determining the mass of the particle by a frequency measurement, said second device comprising at least one gravimetric detector (4) arranged across the electrospray ionization source which is the first device (1), and is fabricated from a layer (7) which also carries the third device (which acts as an electrostatic lens, comprising insulating layer (3) with opening (13) on it,
a third device which is an insulating layer (3) for holding the incoming charges (2) deposited and continuously replenished by the first device (1), with an opening (13), aligned with the second device, through which incoming charges are focused, for obtaining electrostatic lensing effect. Obviously, the layer (7) can be composed of a different material than the material of the substrate 5; or it can be made of the same material with the substrate 5 and is an extension of substrate 5 (it is understood that in this case layer 7 is defined by the micro/nanofabrication process). Moreover, the gravimetric detector 4, the layer 7 and the substrate 5, by themselves or a combination may be formed of many different material layers, such as Silicon-on-Insulator, in other words Silicon-Silicon Dioxide-Silicon.

The substrate (5) is part of a chip that holds both the second device and third device. The chip (composed of 5, 4, 7 and preferably insulating layer 3 with opening 13) sits on a platform (6) which provides mechanical support as well as can have the form of a printed circuit board to interface the gravimetric detector (4) to external electronic instruments.

As charges (2) from the electrospray ionization source which is the first device (1) are accumulated on the insulating layer (3), they create a large electric field towards (for positively charged particles) the opening (13) in the insulating layer (3) which can be implemented by a layer of material with sufficient electrical resistivity, or more specifically the discharging time constant defined by the effective resistance times the effective capacitance to a nearby conductive electrode is long enough so that insulating layer (3) sustains charge on it strong and long enough as to induce electrostatic lensing for incoming particles. The polarity of the charges on insulating layer (3) should be the same with the polarity of the analyte particles as they are electrosprayed. The incoming analyte particles are deflected by the charges (2) on insulating layer (3) and are focused through the opening (13) to be collected efficiently by the gravimetric device. There may be additional layers deposited in between the layer (7) and the insulating layer (3) for instance.

The charges(2) provided by the electrospray ionization source is not necessarily composed entirely by the ions of the analyte particle. Other electrolytes in the solution may also be converted into gas phase ions by the electrospray ionization source, and these ions may also play an instrumental role in sustaining the charge accumulation over the insulating layer (3).

FIG. 2 is a schematic view of a particular embodiment of the device for determining the mass of single nanoparticles, viruses and proteins in a suspension or solution in a fluid with high collection efficiency. Said device comprises:
- a first device (1) which is an electrospray ionization source for electrospraying the fluid to generate charged droplets containing the analyte particles and to obtain a charged flux comprising at least the particle, whereby droplet formation and focusing is facilitated by sheath gas (10) introduced by a tube (15), flow, either nearly parallel or concentric with the electrospray direction, wherein electrospray ionization source and/or charge-generation source (8) deposit and continuously replenish charges (2) which are held on an insulating layer (3) with an opening (13) for obtaining an electrostatic lensing effect,
- a third device which is an insulating layer (3) with an opening (13),
- a lens (12) that shields the gravimetric detector 4 from the adverse effects of the electrospray ionization source, and may provide additional electrostatic lensing of ions, and is composed of either a single conductor or an array of multiple conductor electrodes, e.g. as in an Einzel lens, wherein drying gas (11) introduced by a tube (15), flows above and/or below the lens (12) to facilitate with the evaporation of the droplets,
- a second device comprising at least one gravimetric detector (4) produced on a chip for determining the mass of the particle by a frequency measurement, that gravimetric detector (4) is arranged across the electrospray ionization source, which is the first device (1) and opening of the lens (12),
- a voltage source (9) which applies at the substrate (5) either directly, or through the platform (6) of the chip.

The substrate (5) is part of the chip that holds both the second device comprising at least a gravimetric detector (2) and third device which is insulating layer (3). The chip sits on the platform (6) which provides mechanical support as well as can have the form of a printed circuit board to interface the gravimetric detector (4) to external electronic instruments.

In this embodiment, the addition of the drying gas (11) introduced by a tube (15), increases the rate of evaporation for the charged droplets generated by the electrospray ionization source which is the first device (1). This way, the desolvated analyte ions can be generated at shorter distances with respect to the electrospray ionization source which is the first device (1). As a result, the distance between the electrospray ionization source which is the first device (1) and the gravimetric detector (4) may be decreased for obtaining larger collection efficiencies.

Since gravimetric detectors (4) having enough resolution to measure the mass of nanoparticles and viruses are miniscule, the presence of a nearby electrospray ionization source may cause unwanted effects such as an increase in the noise level, arcing and unintended deposition of large salt crystals or water droplets. The presence of lens (12) is intended to shield the gravimetric detector (4) from such adverse effects. The lens (12) may be placed close to the electrospray ionization source which is the first device (1) as to avoid clipping particles of interest. Moreover, the lens (12) can form as an additional electrostatic lens connected to a voltage source (9) to focus the ions coarsely on the chip. While the lensing effect of lens (12) can provide millimeter scale spot size, the on-chip lensing third device which is insulating layer (3) with an opening (13) aligned with the second device, can provide a focusing spot size on the order of micrometers. Therefore, the lens (12) is seen as an auxiliary mechanism, compared to the critical effect of the third device.

The sheath gas (10) is provided to further focus the electrosprayed microdroplets. The sheath gas (10) can be introduced through a circular and preferably tilted slot concentric with the electrospray ionization source.

A voltage may be applied by a voltage source (9) to the substrate (5) of the chip either directly or through the platform (6) holding the chips, to accelerate and increase the focusing of ions towards the chip (the entire assembly of 5, 7, 4, and preferably 3 with 13), or if desired to decelerate the ions for accomplishing soft landing, in other words the adsorption of material on a surface with minimal chemical and structural changes.

To increase the amount of charges (2) on the insulating layer (3), a charge-generation source (8) different than the original electrospray ionization source which is the first device (1) may be used. The charge-generation source (8) may be another electrospray ionization source, a corona discharge source, an ionizing radiation source such as a radioactive emitter or soft X-ray source, or any other suitable device. The utility of charge-generation source (8) is that the focusing performance of the third device is decoupled from the dynamics and the composition of the electrospray ionization source which is the first device (1) process that generates the particles of interest for detection.

While the embodiments in FIG. 2 depict the chip substrate (5) and the platform (6) as a continuous block of material, another embodiment is envisaged where the substrate (5) and the platform (6) has a small hole just underneath the gravimetric detector (4), through which a suction is provides to further increase the collection efficiency.

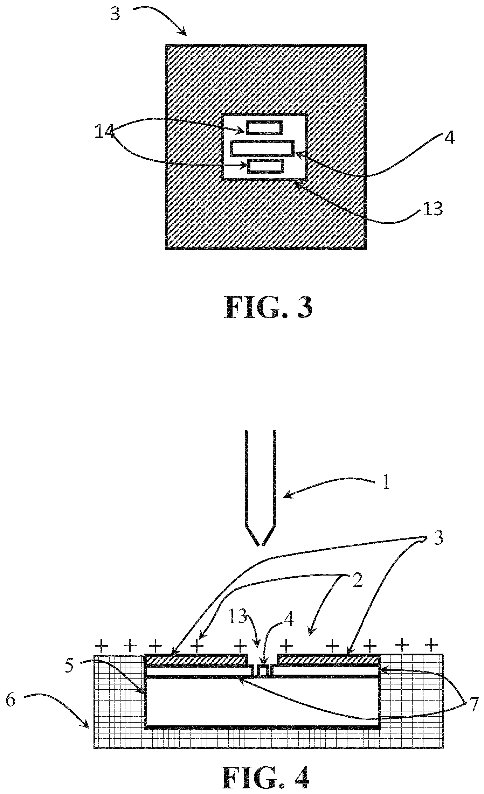

FIG. 3 is a schematic of the top-down view of the chip surface which comprises:
- an insulating layer (3) on which the charges (2) / ions are accumulated to generate the focusing effect,
- an opening (13) on this insulating layer (3) through which the particles of interest pass and are focused, wherein a collection of electrodes (14) are near the gravimetric detector (4) for further focusing the incoming flux within the opening (13),
- a second device comprising at least one gravimetric detector (4) for determining the mass of the particle by a frequency measurement.

FIG. 4 is a schematic view of a particular embodiment of the device for determining the mass of single nanoparticles, viruses and proteins in a suspension or solution in a fluid with high collection efficiency. Said device comprises:
- a first device (1) which is an electrospray ionization source for electro spraying the fluid to generate charged droplets containing the analyte particles and to obtain a charged flux comprising at least the particle,
- a second device comprising at least one gravimetric detector (4) produced on a chip for determining the mass of the particle by a frequency measurement, that gravimetric detector (4) is arranged across the electrospray ionization source, which is the first device (1), and is preferably fabricated on a layer (7) which also carries the insulating layer (3) on it directly or indirectly,
- a third device which is an insulating layer (3) that surrounds the rest of the chip,
- a platform (6) having an insulating top surface and a recessed section into which gravimetric detector (4) can be placed;
   wherein both the platform (6) and the insulating layer (3) are configured to hold accumulated charges (2) deposited and continuously replenished by the first device (1), with an opening (13) for obtaining electrostatic lensing effect.

The substrate (5) is part of the chip that holds both the insulating layer (3) with the opening (13) and the gravimetric detector (4). The chip (composed of 5, 7, 4 and preferably 3 with 13) is situated on the platform (6) with a recess which provides mechanical support as well as can have the form of a printed circuit board to interface the gravimetric detector (4) to external electronic instruments.

In this embodiment of the device, the platform (6) has an insulating top surface and a recessed section into which gravimetric detector (4) can be placed. The advantage of this embodiment is that a smaller chip can be used since the charge accumulation to induce electrostatic charging is performed both by the top surface of the platform (6) and the insulating layer (3). The gap between the platform (6) and the insulating layer (3) may be filled by the application of a suitable insulating filler material. Obviously, the recessed platform (6) can also be used to replace the platform (6) in the embodiment shown in FIG 2.

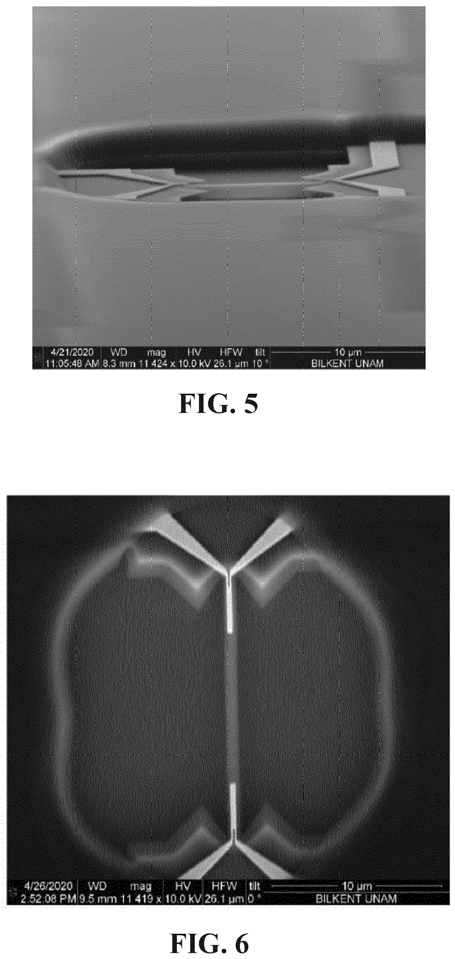

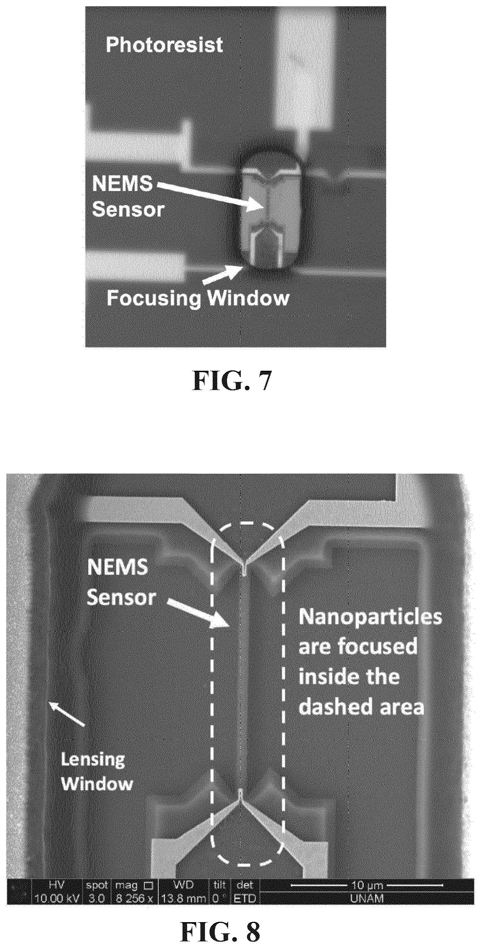

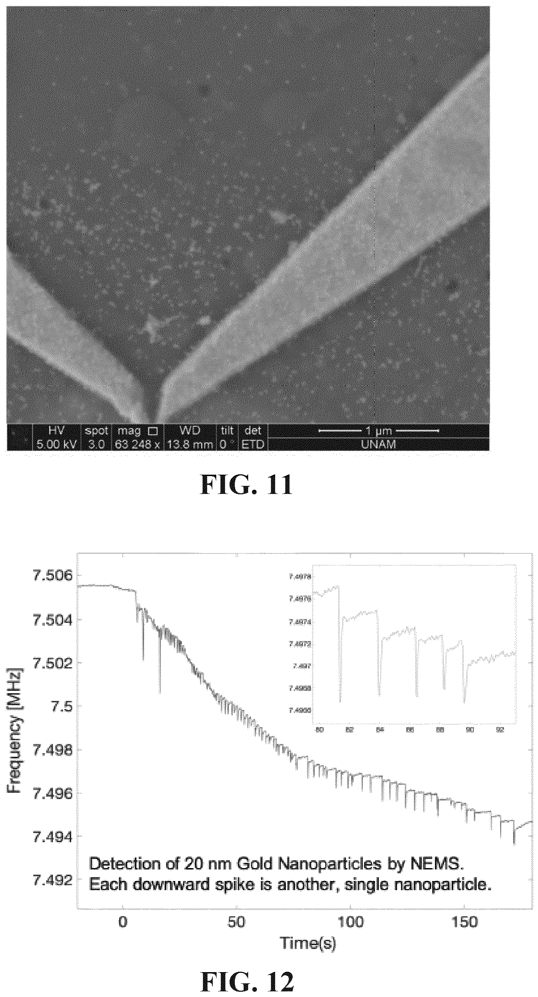

Rapid testing of potential patients before the symptoms appear is still an important problem. It is reported progress towards a microchip-based technology for the detection of SARS-CoV-2 virus at the asymptotic stage. The microchip-based technology is called Nano-Electromechanical Systems (NEMS) and the principle of detection is NEMS-based Mass Spectrometry (NEMS MS). Commercial mass spectrometers cannot directly detect viruses due to their large masses. On the other hand, viruses can easily be detected by the emerging NEMS Mass Spectrometry, with a single-virus resolution. Indeed, during the last decade, it has been already shown that the detection and mass measurement of single biological particles such as BSA (66kDa), IgM (1MDa), bacteriophages (~100MDa). The real challenge with NEMS Mass Spectrometry is the low capture cross-section due to the small size of the sensor. In the present invention, nanoparticles/viruses are generated in the gas phase by Electrospray Ionization (ESI) and then deposited onto a chip patterned with photoresist (Figure 5). Since photoresist is an insulator, the regions covered by it will quickly accumulate charge from the analyte ions arriving initially. The charged photoresist will then act as a highly-localized electrostatic lens that will direct the incoming particles to regions without photoresist. When the photoresist (or another suitable insulating material) covers around the NEMS to form a window above the NEMS plane as shown in Figure 5 and 6, then the analyte particles can effectively be focused on the NEMS device as shown in Figures 7, 8, 9, 10, 11, 12, 13 and 14.

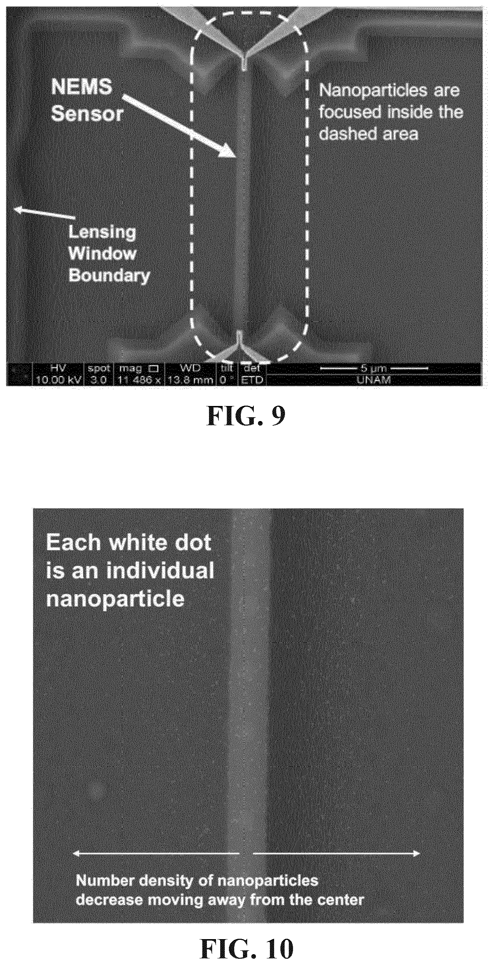

With this "self-lensing" technique, gold and polystyrene nanoparticle have been already delivered onto NEMS with an efficiency better than 1 particle in a million (Figure 10). By fabricating such "lensing windows" in alignment with the tiny NEMS devices on the chip, the majority of the incoming viruses can be transmitted on the NEMS devices. Parallel use of such insulating, self-biased lenses will provide an increase of throughput for sensing based on NEMS arrays.

It is proposed that the use of packaging techniques to cover the bonding pads and wirebonds with an insulating material to increase the throughput of the technique by reducing the analyte losses since these metallic surfaces will also act as electrostatic collectors. The difference between a packaged and unpackaged NEMS device of similar size, collecting an equivalent analyte flux is shown in Figure 14. Clearly packaged devices can collect a larger fraction of the analytes. It is also proposed that the use of backside electrical connections, such as through-silicon-via connections, in conjunction with NEMS sensors to increase the throughput by avoiding front-side wirebonding.

The hydrocarbon chain length in the lipid part of the virion and the number of spike proteins are variable, which will cause a spread in the mass of SARS-CoV-2. For this reason, one of the strategies of the present invention is to measure and identify via the nucleocapsid part of the virus which has a more specific structure. The nucleocapsid is obtained by treating the entire virion with a low-molecular weight, mild detergent (so as not to disrupt the core, but to dissolve the lipid shell).

After obtaining sample, a centrifugation step for pelleting the cells and mucins will be performed first. Introducing a release agent at this stage will facilitate the dissociation of the virions from the cells. The supernatant-rich with background proteins at this stage- will then be buffer exchanged into 10mM ammonium acetate, which is the optimal solution for ESI process. The buffer exchange step will be performed by centrifugal filters with 100kDa molecular weight cutoff, so the majority of the proteins will be separated away from the virus samples. The processed sample will then be used in our NEMS Mass Spectrometer: a large number of hits at the nucleocapsid mass will translate into a positive identification.

It is clear that the proposed invention can similarly be used on processed or natural samples of other viruses, nanoparticles and proteins for the diagnosis of diseases, the characterization of samples e.g. for biomedical screening or pollution monitoring etc.

### REFERENCES

[1] A.K. Naik, et al., "Towards single-molecule nanomechanical mass spectrometry." Nature Nanotechnology, vol. 4. No. 7, XP 055024639, Jun. 21, 2009, pp. 445-450.
[2] M.S. Hanay, et al., "Single-protein nanomechanical mass spectrometry in real time." Nature Nanotechnology, vol. 7, Aug. 26, 2012, pp. 602-608.
[3] S. Dominguez-Medina, et al., "Neutral mass spectrometry of virus capsids above 100 megadaltons with nanomechanical resonators." Science, vol. 362 (6417), Nov. 23, 2018, 918-922
[4] O. Malvar, et al., "Mass and stiffness spectrometry of nanoparticles and whole intact bacteria by multimode nanomechanical resonators." Nature Communications, vol. 7, Nov. 11, 2016, 13452.
[5] J.D. Spitzberg, et al., "Plasmonic-nanopore biosensors for superior single-molecule detection" Advanced Materials, vol. 31, April 3, 2019, 1900422.
[6] Kim, et al, "Parallel patterning of nanoparticles via electrodynamic focusing of charged aerosols", doi:10.1038/nnano.2006.94.

## Claims

1. A device for determining the mass of single nanoparticles, viruses and proteins in suspension or in solution with high-collection efficiency, **characterized by** comprising;
- a first device (1) for creating charged particles of interest in gas phase, wherein the first device (1) is an ionization source,
- a second device for determining the mass of the particle by a frequency measurement comprising at least one gravimetric detector (4) produced on a chip,
- a third device that is fabricated on the same chip with the second device, wherein the third device is an insulating layer (3) surrounding the second device and contains at least one opening (13) aligned with the second device to focus and guide the majority of the incoming charged particles on the second device, by accumulating part of the incoming charges on itself to act as an electrostatic lens.

2. A device according to Claim 1, **characterized by** further comprising a separate electrostatic lens (12) in the free space between the first device (1) and the third device for coarse focusing of the charged particles and shielding the second device from adverse effects of the first device (1).

3. A device according to Claim 1, **characterized in that** the first device (1) is an electrospray ionization source with a tip radius small enough to sustain and electrospray into a low-vacuum chamber in which the first device is housed.

4. A device according to Claim 3, **characterized in that** the first device is selected from electrospray ionization, electrohydrodynamic ionization, matrix assisted laser-desorption ionization (MALDI), laser induced acoustic desorption (LIAD) sources, ultrasonic nebulizer, microwave induced nebulization device, microcapillary array nebulizer, surface acoustic wave nebulizer.

5. A device according to Claim 3, **characterized in that** the first device (1) is supplemented by field emission or corona discharge as an auxiliary technique to introduce additional charges on the droplets.

6. A device according to Claim 1, **characterized in that** the second device and the third devices are situated in a high-vacuum or ultra-high vacuum chamber.

7. A device according to Claim 1, **characterized in that** the gravimetric detector is selected from any of nano-electromechanical systems, micro-electromechanical systems, quartz crystal microbalances, surface acoustic resonators, bulk acoustic resonators, impact detectors or resonant microwave detectors.

8. A device according to Claim 1, **characterized in that** the third device is formed on a layer (7) that is just on the top of a substrate (5) which is a part of the chip that contains the second device.

9. A device according to Claim 8, **characterized in that** the layer (7) is made of any sufficiently insulating material.

10. A device according to Claim 9, **characterized in that** the layer (7) is made of polymers, photoresists, or dielectrics.

11. A device according to Claim 10, **characterized in that** the layer (7) is made of silicon dioxide or silicon nitride.

12. A device according to Claim 1, **characterized in that** the third device has a thickness of 20 nm to 1 mm so that the charges accrued on the third device does not get neutralized quickly.

13. A device for determining the mass of single nanoparticles, viruses and proteins in suspension or in solution with high-collection efficiency, according to Claim 1, **characterized in that**;
- the first device (1) is an electrospray ionization source for electro-spraying a fluid, to generate charged droplets containing the analyte particles and to obtain a charged flux comprising at least the particle,
- the second device comprises at least one gravimetric detector (4) which is arranged across the electrospray ionization source, and is fabricated from a layer (7) which also carries the third device on it,
- the third device which is the insulating layer (3) that surrounds the rest of the chip, wherein the insulating layer (3) is configured to hold accumulated charges (2) deposited and continuously replenished by the first device (1), with the opening (13) for obtaining electrostatic lensing effect,
wherein the device further comprises a substrate (5) which is a part of the chip, that holds both the insulating layer (3) with the opening (13) and gravimetric detector (4).

14. A device according to Claim 13, **characterized in that** the layer (7) is composed of a different material than material of the substrate (5) or it made of the same material with the substrate (5).

15. A device according to Claim 13, **characterized in that** the gravimetric detector (4), the layer (7) and the substrate (5), by themselves or a combination is formed of many different material layers.

16. A device according to Claim 15, **characterized in that** the gravimetric detector (4), the layer (7) and the substrate (5), by themselves or a combination is formed of silicon-on-insulator.

17. A device according to Claim 13, **characterized in that** the chip sits on a platform (6) which provides mechanical support as well as can have the form of a printed circuit board to interface the gravimetric detector (4) to external electronic instruments.

18. A device for determining the mass of single nanoparticles, viruses and proteins in suspension or in solution with high-collection efficiency, according to Claim 13, **characterized in that** droplet formation and focusing is facilitated by sheath gas (10) introduced through a circular and preferably tilted slot concentric with the electrospray ionization source, either flowing nearly parallel or concentric with the electrospray direction, wherein the electrospray ionization source and/or a charge-generation source (8) deposit and continuously replenish charges (2) which are held on the insulating layer (3) with the opening (13) for obtaining an electrostatic lensing effect,
wherein the device further comprises;
- a lens (12) that shields the gravimetric detector (4) from adverse effects of the electrospray ionization source (1), and may provide additional electrostatic lensing of ions, and is composed of either a single conductor or an array of multiple conductor electrodes, wherein the drying gas (11), flows above and/or below the lens (12) to facilitate with the evaporation of the droplets, and
- a voltage source (9) which applies voltage at a substrate (5) either directly or through a carrier platform (6) of the chip wherein the gravimetric detector (4) is arranged across the electrospray ionization source, which is the first device (1), and the opening of the lens (12)

19. A device according to Claim 18, **characterized in that** the chip is a microchip and sits on the platform (6) which provides mechanical support as well as can have the form of a printed circuit board to interface the gravimetric detector to external electronic instruments.

20. A device according to Claim 18, **characterized in that** the lens 12 is placed close to the electrospray ionization source 1 as to avoid clipping particles of interest.

21. A device according to Claim 18, **characterized in that** the lens 12 can be formed as an additional electrostatic lens connected to a voltage source to focus the ions coarsely on the chip.

22. A device for determining the mass of single nanoparticles, viruses and proteins in suspension or in solution with high-collection efficiency, according to Claim 13, **characterized by** further comprising a platform (6) having an insulating top surface and a recessed section into which gravimetric detector (4) can be placed, wherein both the platform (6) and the insulating layer (3) are configured to hold accumulated charges (2) deposited and continuously replenished by the first device (1), with an opening (13) for obtaining an electrostatic lensing effect.

23. A device according to Claim 22, **characterized in that** the chip is situated on the platform (6) with a recess which provides mechanical support as well as can have the form of a printed circuit board to interface the gravimetric detector (4) to external electronic instruments.

24. A device according to Claim 22, **characterized in that** gap between the platform (6) and the insulating layer (3) is filled by an insulating filler material.

25. A device according to Claim 1, wherein the second device is an array of gravimetric sensors, and the third device has multiple openings aligned with the sensors in the array.

## Patentansprüche

1. Vorrichtung zur Bestimmung der Masse einzelner Nanopartikel, Viren und Proteine in Suspension oder in Lösung mit hoher Sammeleffizienz, **dadurch gekennzeichnet, dass** sie umfasst:
- eine erste Vorrichtung (1) zur Erzeugung von geladenen Teilchen von Interesse in der Gasphase, wobei die erste Vorrichtung (1) eine Ionisationsquelle ist,
- eine zweite Vorrichtung zur Bestimmung der Masse des Partikels durch eine Frequenzmessung, die mindestens einen gravimetrischen Detektor (4) umfasst, der auf einem Chip hergestellt ist,
- eine dritte Vorrichtung, die auf demselben Chip wie die zweite Vorrichtung hergestellt ist, wobei die dritte Vorrichtung eine Isolierschicht (3) ist, die die zweite Vorrichtung umgibt und mindestens eine Öffnung (13) enthält, die mit der zweiten Vorrichtung ausgerichtet ist, um den Großteil der einfallenden geladenen Teilchen auf die zweite Vorrichtung zu fokussieren und zu leiten, indem sie einen Teil der einfallenden Ladungen auf sich selbst akkumuliert, um als elektrostatische Linse zu wirken.

2. Vorrichtung nach Anspruch 1, **dadurch gekennzeichnet, dass** sie ferner eine separate elektrostatische Linse (12) im freien Raum zwischen der ersten Vorrichtung (1) und der dritten Vorrichtung zum Grobfokussieren der geladenen Teilchen und zum Abschirmen der zweiten Vorrichtung vor nachteiligen Einflüssen der ersten Vorrichtung (1) umfasst.

3. Vorrichtung nach Anspruch 1, **dadurch gekennzeichnet, dass** die erste Vorrichtung (1) eine Elektrospray-Ionisationsquelle mit einem Spitzenradius ist, der klein genug ist, um in einer Niedervakuumkammer, in der die erste Vorrichtung untergebracht ist, aufrechtzuerhalten und in diese hinein zu elektrosprayen.

4. Vorrichtung nach Anspruch 3, **dadurch gekennzeichnet, dass** die erste Vorrichtung ausgewählt ist aus Elektrospray-Ionisation, elektrohydrodynamischer Ionisation, Matrix-unterstützter Laser-Desorptionsionisation (MALDI), Laser-induzierter akustischer Desorption (LIAD), Ultraschallvernebler, mikrowelleninduzierter Vernebelungsvorrichtung, Mikrokapillar-Array-Vernebler, Oberflächenwellenvernebler.

5. Vorrichtung nach Anspruch 3, **dadurch gekennzeichnet, dass** die erste Vorrichtung (1) durch Feldemission oder Koronaentladung als Hilfstechnik ergänzt ist, um zusätzliche Ladungen auf die Tröpfchen einzubringen.

6. Vorrichtung nach Anspruch 1, **dadurch gekennzeichnet, dass** die zweite Vorrichtung und die dritte Vorrichtung in einer Hochvakuum- oder Ultrahochvakuumkammer angeordnet sind.

7. Vorrichtung nach Anspruch 1, **dadurch gekennzeichnet, dass** der gravimetrische Detektor aus Nanoelektromechanischen Systemen, Mikroelektromechanischen Systemen, Quarzkristall-Mikrowaagen, Oberflächenakustikresonatoren, Volumenakustikresonatoren, Aufpralldetektoren oder Resonanzmikrowellendetektoren ausgewählt ist.

8. Vorrichtung nach Anspruch 1, **dadurch gekennzeichnet, dass** die dritte Vorrichtung auf einer Schicht (7) ausgebildet ist, die sich direkt auf einem Substrat (5) befindet, das Teil des Chips ist, der die zweite Vorrichtung enthält.

9. Vorrichtung nach Anspruch 8, **dadurch gekennzeichnet, dass** die Schicht (7) aus einem beliebigen ausreichend isolierenden Material besteht.

10. Vorrichtung nach Anspruch 9, **dadurch gekennzeichnet, dass** die Schicht (7) aus Polymeren, Fotolacken oder Dielektrika besteht.

11. Vorrichtung nach Anspruch 10, **dadurch gekennzeichnet, dass** die Schicht (7) aus Siliziumdioxid oder Siliziumnitrid besteht.

12. Vorrichtung nach Anspruch 1, **dadurch gekennzeichnet, dass** die dritte Vorrichtung eine Dicke von 20 nm bis 1 mm aufweist, so dass die auf der dritten Vorrichtung aufgeladenen Ladungen nicht schnell neutralisiert werden.

13. Vorrichtung zur Bestimmung der Masse einzelner Nanopartikel, Viren und Proteine in Suspension oder Lösung mit hoher Sammeleffizienz, gemäß Anspruch 1, **dadurch gekennzeichnet, dass**
- die erste Vorrichtung (1) eine Elektrospray-Ionisationsquelle zum Elektrosprayen eines Fluids ist, um geladene Tröpfchen zu erzeugen, die die Analytpartikel enthalten, und um einen geladenen Fluss zu erhalten, der mindestens die Partikel umfasst,
- die zweite Vorrichtung mindestens einen gravimetrischen Detektor (4) umfasst, der quer zur Elektrospray-Ionisationsquelle angeordnet ist und aus einer Schicht (7) hergestellt ist, die auch die dritte Vorrichtung trägt,
- die dritte Vorrichtung die Isolierschicht (3) ist, die den Rest des Chips umgibt, wobei die Isolierschicht (3) so konfiguriert ist, dass sie angesammelte Ladungen (2) hält, die von der ersten Vorrichtung (1) abgeschieden und kontinuierlich aufgefüllt werden, wobei die Öffnung (13) dazu dient, einen elektrostatischen Linseneffekt zu erzielen,
wobei die Vorrichtung ferner ein Substrat (5) umfasst, das Teil des Chips ist und sowohl die Isolierschicht (3) mit der Öffnung (13) als auch den gravimetrischen Detektor (4) hält.

14. Vorrichtung nach Anspruch 13, **dadurch gekennzeichnet, dass** die Schicht (7) aus einem anderen Material als dem Material des Substrats (5) besteht oder aus dem gleichen Material wie das Substrat (5) hergestellt ist.

15. Vorrichtung nach Anspruch 13, **dadurch gekennzeichnet, dass** der gravimetrische Detektor (4), die Schicht (7) und das Substrat (5) allein oder in Kombination aus vielen verschiedenen Materialschichten gebildet sind.

16. Vorrichtung nach Anspruch 15, **dadurch gekennzeichnet, dass** der gravimetrische Detektor (4), die Schicht (7) und das Substrat (5) allein oder in Kombination aus Silizium-auf-Isolator gebildet sind.

17. Vorrichtung nach Anspruch 13, **dadurch gekennzeichnet, dass** der Chip auf einer Plattform (6) sitzt, die mechanische Unterstützung bietet und die Form einer Leiterplatte haben kann, um den gravimetrischen Detektor (4) mit externen elektronischen Instrumenten zu verbinden.

18. Vorrichtung zur Bestimmung der Masse einzelner Nanopartikel, Viren und Proteine in Suspension oder in Lösung mit hoher Sammeleffizienz, gemäß Anspruch 13, **dadurch gekennzeichnet, dass** die Tröpfchenbildung und Fokussierung durch Hüllgas (10) erleichtert wird, das durch einen kreisförmigen und vorzugsweise geneigten Schlitz konzentrisch zur Elektrospray-Ionisationsquelle entweder nahezu parallel oder konzentrisch zur Elektrospray-Richtung strömt, wobei die Elektrospray-Ionisationsquelle und/oder eine Ladungserzeugungsquelle (8) Ladungen (2) ablegen und kontinuierlich auffüllen, die auf der Isolierschicht (3) mit der Öffnung (13) gehalten werden, um einen elektrostatischen Linseneffekt zu erzielen.
wobei die Vorrichtung ferner umfasst:
- eine Linse (12), die den gravimetrischen Detektor (4) vor nachteiligen Einflüssen der Elektrospray-Ionisationsquelle (1) abschirmt und eine zusätzliche elektrostatische Linsenwirkung für Ionen bereitstellen kann und entweder aus einem einzelnen Leiter oder einer Anordnung mehrerer Leiterelektroden besteht, wobei das Trocknungsgas (11) über und/oder unter der Linse (12) strömt, um die Verdampfung der Tröpfchen zu erleichtern, und
- eine Spannungsquelle (9), die Spannung entweder direkt oder über eine Trägerplattform (6) des Chips an ein Substrat (5) anlegt, wobei der gravimetrische Detektor (4) über der Elektrospray-Ionisationsquelle, die die erste Vorrichtung (1) ist, und der Öffnung der Linse (12) angeordnet ist.

19. Vorrichtung nach Anspruch 18, **dadurch gekennzeichnet, dass** der Chip ein Mikrochip ist und auf der Plattform (6) sitzt, die mechanische Unterstützung bietet und die Form einer Leiterplatte haben kann, um den gravimetrischen Detektor mit externen elektronischen Instrumenten zu verbinden.

20. Vorrichtung nach Anspruch 18, **dadurch gekennzeichnet, dass** die Linse 12 nahe an der Elektrospray-Ionisationsquelle 1 angeordnet ist, um ein Abschneiden interessanter Partikel zu vermeiden.

21. Vorrichtung nach Anspruch 18, **dadurch gekennzeichnet, dass** die Linse 12 als zusätzliche elektrostatische Linse ausgebildet sein kann, die mit einer Spannungsquelle verbunden ist, um die Ionen grob auf den Chip zu fokussieren.

22. Vorrichtung zur Bestimmung der Masse einzelner Nanopartikel, Viren und Proteine in Suspension oder in Lösung mit hoher Sammeleffizienz, gemäß Anspruch 13, **dadurch gekennzeichnet, dass** sie ferner eine Plattform (6) mit einer isolierenden Oberseite und einem vertieften Abschnitt umfasst, in den ein gravimetrischer Detektor (4) eingesetzt werden kann, wobei sowohl die Plattform (6) als auch die Isolierschicht (3) so konfiguriert sind, dass sie angesammelte Ladungen (2) aufnehmen, die von der ersten Vorrichtung (1) abgeschieden und kontinuierlich wieder aufgefüllt werden, mit einer Öffnung (13) zum Erzeugen eines elektrostatischen Linseneffekts.

23. Vorrichtung nach Anspruch 22, **dadurch gekennzeichnet, dass** der Chip auf der Plattform (6) mit einer Aussparung angeordnet ist, die mechanische Abstützung bietet und die Form einer Leiterplatte haben kann, um den gravimetrischen Detektor (4) mit externen elektronischen Instrumenten zu verbinden.

24. Vorrichtung nach Anspruch 22, **dadurch gekennzeichnet, dass** der Spalt zwischen der Plattform (6) und der Isolierschicht (3) mit einem isolierenden Füllmaterial gefüllt ist.

25. Vorrichtung nach Anspruch 1, wobei die zweite Vorrichtung eine Anordnung von gravimetrischen Sensoren ist und die dritte Vorrichtung mehrere Öffnungen aufweist, die mit den Sensoren in der Anordnung ausgerichtet sind.

## Revendications

1. Dispositif permettant de déterminer la masse de nanoparticules, de virus et de protéines individuels en suspension ou en solution avec une efficacité de collecte élevée, **caractérisé en ce qu'**il comprend ;
- un premier dispositif (1) de création de particules chargées d'intérêt en phase gazeuse, dans lequel le premier dispositif (1) est une source d'ionisation ;
- un second dispositif de détermination de la masse de la particule par mesure de fréquence, comprenant au moins un détecteur gravimétrique (4) réalisé sur une puce.
- un troisième dispositif qui est fabriqué sur la même puce que le deuxième dispositif, dans lequel le troisième dispositif est une couche isolante (3) entourant le deuxième dispositif et contient au moins une ouverture (13) alignée avec le deuxième dispositif pour focaliser et guider la majorité des particules chargées entrantes sur le deuxième dispositif, en accumulant une partie des charges entrantes sur lui-même pour agir comme une lentille électrostatique.

2. Dispositif selon la revendication 1, **caractérisé en ce qu'**il comprend en outre une lentille électrostatique séparée (12) dans l'espace libre entre le premier dispositif (1) et le troisième dispositif pour une focalisation grossière des particules chargées et pour protéger le deuxième dispositif des effets indésirables du premier dispositif (1).

3. Dispositif selon la revendication 1, **caractérisé en ce que** le premier dispositif (1) est une source d'ionisation par électrospray avec un rayon de pointe suffisamment petit pour maintenir un électrospray dans une chambre à vide faible dans laquelle le premier dispositif est logé.

4. Dispositif selon la revendication 3, **caractérisé en ce que** le premier dispositif est choisi parmi l'ionisation par électrospray, l'ionisation électrohydrodynamique, l'ionisation par désorption laser assistée par matrice (MALDI), les sources de désorption acoustique induite par laser (LIAD), le nébuliseur à ultrasons, le dispositif de nébulisation induite par micro-ondes, le nébuliseur à réseau microcapillaire, le nébuliseur à ondes acoustiques de surface.

5. Dispositif selon la revendication 3, **caractérisé en ce que** le premier dispositif (1) est complété par une émission de champ ou une décharge corona comme technique auxiliaire pour introduire des charges supplémentaires sur les gouttelettes.

6. Dispositif selon la revendication 1, **caractérisé en ce que** le deuxième dispositif et le troisième dispositif sont situés dans une chambre à vide élevé ou à ultravide.

7. Dispositif selon la revendication 1, **caractérisé en ce que** le détecteur gravimétrique est choisi parmi l'un quelconque des systèmes nano-électromécaniques, des systèmes micro-électromécaniques, des microbalances à cristal de quartz, des résonateurs acoustiques de surface, des résonateurs acoustiques en volume, des détecteurs d'impact ou des détecteurs micro-ondes résonants.

8. Dispositif selon la revendication 1, **caractérisé en ce que** le troisième dispositif est formé sur une couche (7) qui se trouve juste au-dessus d'un substrat (5) qui fait partie de la puce qui contient le deuxième dispositif.

9. Dispositif selon la revendication 8, **caractérisé en ce que** la couche (7) est réalisée en tout matériau suffisamment isolant.

10. Dispositif selon la revendication 9, **caractérisé en ce que** la couche (7) est constituée de polymères, de résines photosensibles ou de diélectriques.

11. Dispositif selon la revendication 10, **caractérisé en ce que** la couche (7) est en dioxyde de silicium ou en nitrure de silicium.

12. Dispositif selon la revendication 1, **caractérisé en ce que** le troisième dispositif a une épaisseur de 20 nm à 1 mm de sorte que les charges accumulées sur le troisième dispositif ne soient pas neutralisées rapidement.

13. Dispositif de détermination de la masse de nanoparticules, de virus et de protéines individuels en suspension ou en solution avec une efficacité de collecte élevée, selon la revendication 1, **caractérisé en ce que** ;
- le premier dispositif (1) est une source d'ionisation par électrospray pour électropulvériser un fluide, pour générer des gouttelettes chargées contenant les particules d'analyte et pour obtenir un flux chargé comprenant au moins la particule,
- le deuxième dispositif comprend au moins un détecteur gravimétrique (4) qui est disposé en travers de la source d'ionisation par électrospray, et est fabriqué à partir d'une couche (7) qui porte également le troisième dispositif sur elle,
- le troisième dispositif qui est la couche isolante (3) qui entoure le reste de la puce, dans laquelle la couche isolante (3) est configurée pour contenir les charges accumulées (2) déposées et continuellement renouvelées par le premier dispositif (1), avec l'ouverture (13) pour obtenir un effet de lentille électrostatique,
dans lequel le dispositif comprend en outre un substrat (5) qui fait partie de la puce, qui contient à la fois la couche isolante (3) avec l'ouverture (13) et le détecteur gravimétrique (4).

14. Dispositif selon la revendication 13, **caractérisé en ce que** la couche (7) est composée d'un matériau différent de celui du substrat (5) ou est constituée du même matériau que le substrat (5).

15. Dispositif selon la revendication 13, **caractérisé en ce que** le détecteur gravimétrique (4), la couche (7) et le substrat (5), seuls ou en combinaison, sont formés de nombreuses couches de matériaux différents.

16. Dispositif selon la revendication 15, **caractérisé en ce que** le détecteur gravimétrique (4), la couche (7) et le substrat (5), seuls ou en combinaison, sont constitués de silicium sur isolant.

17. Dispositif selon la revendication 13, **caractérisé en ce que** la puce repose sur une plate-forme (6) qui fournit un support mécanique et peut également avoir la forme d'une carte de circuit imprimé pour interfacer le détecteur gravimétrique (4) avec des instruments électroniques externes.

18. Dispositif de détermination de la masse de nanoparticules, de virus et de protéines individuels en suspension ou en solution avec une efficacité de collecte élevée, selon la revendication 13, **caractérisé en ce que** la formation et la focalisation des gouttelettes sont facilitées par un gaz de gaine (10) introduit à travers une fente circulaire et de préférence inclinée concentrique à la source d'ionisation par électrospray, s'écoulant soit presque parallèlement soit concentriquement à la direction de l'électrospray, dans lequel la source d'ionisation par électrospray et/ou une source de génération de charges (8) déposent et réapprovisionnent en continu des charges (2) qui sont maintenues sur la couche isolante (3) avec l'ouverture (13) pour obtenir un effet de lentille électrostatique,
dans lequel le dispositif comprend en outre ;
- une lentille (12) qui protège le détecteur gravimétrique (4) des effets indésirables de la source d'ionisation par électrospray (1), et qui peut fournir une lentille électrostatique supplémentaire des ions, et qui est composée soit d'un seul conducteur, soit d'un réseau d'électrodes à conducteurs multiples, dans lequel le gaz de séchage (11) circule au-dessus et/ou en dessous de la lentille (12) pour faciliter l'évaporation des gouttelettes, et
- une source de tension (9) qui applique une tension à un substrat (5) soit directement, soit via une plate-forme de support (6) de la puce dans laquelle le détecteur gravimétrique (4) est disposé entre la source d'ionisation par électrospray, qui est le premier dispositif (1), et l'ouverture de la lentille (12).

19. Dispositif selon la revendication 18, **caractérisé en ce que** la puce est une micropuce et repose sur la plate-forme (6) qui fournit un support mécanique et peut également avoir la forme d'une carte de circuit imprimé pour interfacer le détecteur gravimétrique avec des instruments électroniques externes.

20. Dispositif selon la revendication 18, **caractérisé en ce que** la lentille (12) est placée à proximité de la source d'ionisation par électrospray (1) de manière à éviter de couper les particules d'intérêt.

21. Dispositif selon la revendication 18, **caractérisé en ce que** la lentille (12) peut être réalisée sous forme de lentille électrostatique supplémentaire connectée à une source de tension pour focaliser grossièrement les ions sur la puce.

22. Dispositif de détermination de la masse de nanoparticules, de virus et de protéines individuels en suspension ou en solution avec une efficacité de collecte élevée, selon la revendication 13, **caractérisé en ce qu'**il comprend en outre une plate-forme (6) ayant une surface supérieure isolante et une section en retrait dans laquelle un détecteur gravimétrique (4) peut être placé, où la plate-forme (6) et la couche isolante (3) sont toutes deux configurées pour contenir des charges accumulées (2) déposées et continuellement renouvelées par le premier dispositif (1), avec une ouverture (13) pour obtenir un effet de lentille électrostatique.

23. Dispositif selon la revendication 22, **caractérisé en ce que** la puce est située sur la plate-forme (6) avec un évidement qui fournit un support mécanique et peut également avoir la forme d'une carte de circuit imprimé pour interfacer le détecteur gravimétrique (4) avec des instruments électroniques externes.

24. Dispositif selon la revendication 22, **caractérisé en ce que** l'espace entre la plate-forme (6) et la couche isolante (3) est rempli par un matériau de remplissage isolant.

25. Dispositif selon la revendication 1, dans lequel le deuxième dispositif est un réseau de capteurs gravimétriques, et le troisième dispositif comporte de multiples ouvertures alignées avec les capteurs du réseau.
